# EUROPÄISCHE PATENTANMELDUNG

(11) **EP 4 198 123 A1**
(43) Veröffentlichungstag der Anmeldung: **21.06.2023**
(21) Anmeldenummer: 21215753.1
(22) Anmeldetag: 17.12.2021
(51) Int. Cl.: C12N 9/10, C12N 15/52, C12P 13/22

(54) **BESONDERS GEEIGNETE 3-DESOXYARABINOHEPTULOSANAT-7-PHOSPHAT-SYNTHASE ZUR FERMENTATIVEN HERSTELLUNG VON ORTHO-AMINOBENZOESÄURE**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die vorliegende Erfindung betrifft die Verwendung spezieller Varianten der 3-Desoxyarabinoheptulosanat-7-phosphat-Synthase zur Herstellung von ortho-Aminobenzoesäure durch eine mikrobielle Fermentation und Mikroorganismen, die hierfür geeignet sind.

## Beschreibung

Die vorliegende Erfindung betrifft die Verwendung spezieller Varianten der 3-Desoxyarabinoheptulosanat-7-phosphat-Synthase zur Herstellung von ortho-Aminobenzoesäure durch eine mikrobielle Fermentation und Mikroorganismen, die hierfür geeignet sind.

Die Herstellung von ortho-Aminobenzoesäure (oAB) mit Hilfe einer Reihe von genetisch veränderten Mikroorganismen ist aus dem Stand der Technik bekannt, z.B. aus Balderas-Hernandez et al. (2009) "Metabolic engineering for improving anthranilate synthesis from glucose in Escherichia coli", WO 2015/124687 und WO 2017/102853.

Aus EP 0 077 196 ist eine Variante der 3-Desoxyarabinoheptulosanat-7-phosphat-Synthase bekannt, deren Aktivität in Anwesenheit aromatischer Aminosäuren weniger stark inhibiert wird als dies bei anderen Varianten dieses Enzyms der Fall ist.

In der Studie, die der vorliegenden Patentanmeldung zugrunde liegt, wurde überraschend herausgefunden, dass *Corynebacterium glutamicum* in Anwesenheit der in EP 0 077 196 beschriebenen DAHP-Synthase-Variante mehr oAB produziert als dies bei Verwendung anderer Enzymvarianten der Fall ist. Diese Erfindung und vorteilhafte Ausführungsformen davon werden in den Patentansprüchen und nachfolgend in der Beschreibung näher definiert.

Die vorliegende Erfindung betrifft in einer ersten Ausführungsform die Verwendung einer 3-Desoxyarabinoheptulosanat-7-phosphat-Synthase (DAHP-Synthase) mit einer Aminosäuresequenz wie durch SEQ ID NO. 11 definiert oder einer Variante davon zur Herstellung von ortho-Aminobenzoesäure (oAB) durch eine mikrobielle Fermentation.

### Verwendung

Die "Verwendung" der 3-Desoxyarabinoheptulosanat-7-phosphat-Synthase (DAHP-Synthase) mit einer Aminosäuresequenz wie durch SEQ ID NO. 11 definiert besteht darin, dass dieses Enzym in wenigstens einer mikrobiellen Zelle rekombinant exprimiert wird und dass diese wenigstens eine mikrobielle Zelle, die besagtes Enzym rekombinant exprimiert, unter Bedingungen kultiviert wird, die eine metabolische Aktivität der Zelle ermöglichen.

Die erfindungsgemäße Verwendung der DAHP-Synthase wie durch SEQ ID NO. 11 definiert schließt nicht aus, dass die mikrobielle Zelle zusätzlich weitere genetische Modifikationen enthält, insbesondere weitere Proteine rekombinant exprimiert. Diese Modifikationen sind vorzugsweise jene, die weiter unten in dieser Anmeldung offenbart werden.

Da die verbesserte 3-Desoxyarabinoheptulosanat-7-phosphat-Synthase den Fluss von Metaboliten durch den gesamten Shikimat-Weg bis zur oAB erhöht, wird die erfindungsgemäße verbesserte DAHP-Synthase in einer bevorzugten Ausführungsform der vorliegenden Erfindung zur Synthese wenigstens einer Verbindung ausgewählt aus der Gruppe bestehend aus 3-Desoxyarabinoheptulosanat-7-phosphat, 3-Dehydrochinat, 3-Dehydroxyshikimat, Shikimat, Shikimat-3-Phosphat, Chorismat, Prephenat und ortho-Aminobenzoesäure durch eine mikrobielle Fermentation verwendet.

Alle unten in dieser Anmeldung gegebenen Definitionen gelten auch für diese Ausführungsform. Der Fachmann versteht, dass die metabolische und genetische Ausstattung der verwendeten mikrobiellen Zellen oder Zellen an das angestrebte angepasst werden muss, indem die Aktivität von Enzymen, welche die Umsetzung des angestrebten Produkts zu Folgeprodukten katalysieren, reduziert oder ganz unterbunden wird.

### 3-Desoxyarabi noheptul osanat-7-phosphat-Synthase

Der Begriff "3-Desoxyarabinoheptulosanat-7-phosphat-Synthase" bezeichnet das Enzym, welches die erste Reaktion des Shikimisäurewegs katalysiert, nämlich die Umsetzung von Erythrose-4-Phosphat und Phosphoenolpyruvat zu 3-Desoxyarabinoheptulosanat-7-phosphat und einem anorganischen Phosphat. Die Enzymvariante, die eine Aminosäuresequenz gemäß SEQ ID NO. 11 hat, wurde in EP 0 077 196 offenbart.

Eine "Variante" der DAHP-Synthase wie in SEQ ID NO. 11 definiert ein Enzym, das durch Hinzufügung, Deletion oder Austausch von bis zu 5 %, bevorzugt bis zu 2 %, der im jeweiligen Enzym enthaltenen Aminosäuren erhalten wird, mit der Maßgabe, dass die Positionen 76 und 211 unverändert bleiben. Es ist weiterhin bevorzugt, dass zusätzlich auch die Positionen 10, 13, 147, 148, 150, 151, 179, 209, 211 und 212 unverändert bleiben. In einer stärker bevorzugten Ausführungsform der vorliegenden Erfindung sind in der Variante zusätzlich auch die Positionen 144, 175 und 215 unverändert. In einer noch stärker bevorzugten Ausführungsform der vorliegenden Erfindung sind zusätzlich zu den vorgenannten Positionen auch die Positionen 92, 97, 165, 186 und 268 unverändert. Der Fachmann versteht, dass sich die vorgenannten Positionen entsprechend verschieben, wenn Aminosäuren deletiert oder eingefügt werden. Grundsätzlich können die vorgenannten Modifikationen an jeder beliebigen Stelle des Enzyms kontinuierlich oder diskontinuierlich erfolgen. Bevorzugt erfolgen sie aber nur am N-Terminus und/oder am C-Terminus des Polypeptids. Bei Substitutionen von Aminosäuren handelt es sich vorzugsweise um konservative Substitutionen, d.h. um solche, bei denen die veränderte Aminosäure einen Rest mit ähnlichen chemischen Eigenschaften besitzt wie die im unveränderten Enzym vorliegende Aminosäure. Es werden also besonders bevorzugt Aminosäuren mit basischen Resten gegen solche mit basischen Resten, Aminosäuren mit sauren Resten gegen solche mit ebenfalls sauren Resten, Aminosäuren mit polaren Resten gegen solche mit polaren Resten und Aminosäuren mit unpolaren gegen solche mit unpolaren Resten ausgetauscht. Es ist besonders bevorzugt, dass auch die Variante der DAHP-Synthase eine entsprechende Enzymaktivität besitzt. Besonders bevorzugt liegt die spezifische Enzymaktivität der Variante bei wenigstens bei 80 % spezifischen Aktivität der unveränderten DAHP-Synthase gemäß SEQ ID NO. 11.

### Ortho-Aminobenzoesäure

Der Begriff oAB bezeichnet die ortho-Aminobenzoesäure, auch unter der Bezeichnung "Anthranilsäure" bekannt. Da diese Verbindung sowohl über eine Carboxylgruppe mit sauren Eigenschaften als auch über eine Aminogruppe mit basischen Eigenschaften verfügt, ist die Ladung des Moleküls abhängig vom pH. Im Rahmen der vorliegenden Anmeldung bezeichnet der Begriff "ortho-Aminobenzoesäure" das Molekül unabhängig davon, ob es eine positive, eine negative oder gar keine Nettoladung hat.

### Mikrobielle Fermentation

Der Begriff "mikrobielle Fermentation" bezeichnet die Inkubation einer oder mehrerer mikrobieller Zellen in einem geeigneten Nährmedium unter Bedingungen, welche die Stoffwechselaktivität der Zellen ermöglichen. Dies bedeutet insbesondere die Einstellung und Aufrechterhaltung eines geeigneten pH-Wertes, einer geeigneten Sauerstoffsättigung, einer geeigneten Temperatur und einer geeigneten Osmolarität. Der Fachmann kann Kultivierungsbedingungen, die für den jeweiligen Mikroorganismus geeignet sind, anhand seiner Fachkenntnisse und der allgemein verfügbaren Literatur auswählen. Die Kultivierung von *Corynebacterium,* insbesondere *Corynebacterium glutamicum* erfolgt vorzugsweise bei einem pH zwischen 6 und 8, bei einer Temperatur zwischen 25 °C und 40 °C sowie bei einer Osmolalität zwischen 400 und 2600 mOsmol/kg. Die Sauerstoffsättigung wird vorzugsweise möglichst nahe an dem unter Normaldruck und Normalatmosphäre erreichbaren Maximum gehalten.

Insbesondere wird den mikrobiellen Zellen bei einer mikrobiellen Fermentation auch wenigstens eine fermentierbare Kohlenstoffquelle zur Verfügung gestellt. Hierbei handelt es sich um eine organische Kohlenstoffverbindung, die von den Zellen zur Bildung von oAB genutzt werden kann. Es ist bevorzugt, dass sie zusätzlich auch als Energiequelle für den Stoffwechsel sowie als Kohlenstoffquelle für den Aufbau von mikrobieller Biomasse genutzt werden kann. In einer Ausführungsform der vorliegenden Erfindung sind die Stoffwechselwege der mikrobiellen Zelle für die Bildung von mikrobieller Biomasse und die Bildung von oAB soweit getrennt, dass wenigstens eine fermentierbare Kohlenstoffquelle für den Energie- und Baustoffwechsel verwendet wird und wenigstens eine andere für die Produktbildung. Bevorzugte fermentierbare Kohlenstoffquellen sind ausgewählt aus der Gruppe bestehend aus C-5-Monosacchariden, C-6-Monosacchariden, Disacchariden und Trisacchariden. Bevorzugte C-5-Monosaccharide sind Xylose und Arabinose. Bevorzugte C-6-Monosaccharide sind Glucose, Fructose und Mannose. Ein bevorzugtes Disaccharid ist Saccharose. Ein bevorzugtes Trisaccharid ist Kestose.

Die metabolische Aktivität besteht vorzugsweise in der Produktion von oAB und/oder dem Aufbau von Biomasse, besonders bevorzugt in der Produktion von oAB. Ein geeigneter Parameter, der die kontinuierliche Überwachung der metabolischen Aktivität zur Prozesssteuerung erlaubt, ist auch die Sauerstoffaufnahmerate. Diese kann, z.B. durch Sauerstoffelektroden, kontinuierlich erfolgen und liefert ohne zeitlichen Verzug Messergebnisse, die zur Anpassung der Kultivierungsbedingungen verwendet werden können.

### Nährmedium

Nährmedien, die zur Kultivierung mikrobieller Zellen, insbesondere von *Corynebacterium sp.,* geeignet sind, sind aus dem Stand der Technik bekannt. Geeignete Nährmedien enthalten wenigstens einen Puffer zur Regulation des pH, für den verwendeten Mikroorganismus verwertbare Quellen anorganischer Nährstoffe, insbesondere Stickstoff, Schwefel und Phosphor sowie die vom eingesetzten Organismus benötigten Spurenelemente. Je nach Mikroorganismus kann der Zusatz von Vitaminen und/oder komplexen Medienbestandteilen wie z.B. Pepton oder Hefeextrakt sinnvoll sein. Besonders geeignete Nährmedien sind in den in dieser Anmeldung enthaltenen Ausführungsbeispielen beschrieben.

### Mikrobielle Zelle

Bei der mikrobiellen Zelle handelt es sich bevorzugt um eine Bakterienzelle, stärker bevorzugt um einen Stamm des Genus *Corynebacterium* und noch stärker bevorzugt um *Corynebacterium glutamicum.* Wenn im Kontext dieser Anmeldung von "einer mikrobiellen Zelle" die Rede ist, so ist eine Mehrzahl von Zellen immer eingeschlossen, da die Bildung einer nennenswerten Produktmenge in überschaubaren Zeiträumen die Anwesenheit einer Vielzahl von Zellen voraussetzt. Außerdem führt die Kultivierung einer Zelle im Regelfall auch zur Vermehrung der Zelle, so dass selbst bei der Anwesenheit einer einzigen Zelle im Nährmedium nach einiger Zeit eine Mehrzahl von Zellen entsteht.

Bei der mikrobiellen Zelle handelt es sich um eine rekombinante mikrobielle Zelle. Dies bedeutet, dass diese Zelle wenigstens eine Nukleinsäuresequenz enthält, die für ein Gen kodiert, das in der fraglichen Zelle endogen nicht vorhanden ist ("Fremdgen"). Bei diesem Fremdgen kann es sich auch um die Variante eines endogen vorkommenden Enzyms handeln, die sich vom endogen vorkommenden Enzym nur durch eine höhere oder niedrigere Enzymaktivität unterscheidet oder die einem anderen Regulationsmechanismus unterliegt. Dieses Fremdgen muss in einer Form vorliegen, die seine Expression ermöglicht. Dies bedeutet insbesondere, dass es unter Kontrolle eines für die jeweilige mikrobielle Zelle geeigneten Promotors steht. In einer Ausführungsform der vorliegenden Erfindung liegt das Fremdgen mit der für den betreffenden Mikroorganismus geeigneten Promotorsequenz in einem Plasmid vor. In einer anderen Ausführungsform ist das Fremdgen mit der für den betreffenden Mikroorganismus geeigneten Promotorsequenz in das Genom der mikrobiellen Zellen integriert. Obligatorisch enthält die rekombinante mikrobielle Zelle ein Gen, das für eine DAHP-Synthase mit einer Sequenz wie durch SEQ ID NO: 11 definiert oder eine Variante davon kodiert.

In einer bevorzugten Ausführungsform der vorliegenden Erfindung handelt es sich bei der mikrobiellen Zelle um eine Zelle, die sich zusätzlich zur Anwesenheit der oben definierten DAHP-Synthase gemäß SEQ ID NO. 11 oder einer Variante davon vom Wildtyp des betreffenden Mikroorganismus wenigstens durch folgende Merkmale unterscheidet:
(i) Eine gegenüber dem jeweiligen Wildtyp reduzierte Aktivität der Anthranilat-Phosphoribosyltransferase, wobei aber eine Restaktivät vorhanden sein muss. Die Restaktivität liegt vorzugsweise zwischen 10 % und 60 %, stärker bevorzugt zwischen 20 % und 50 % der in *C. glutamicum* ATCC13032 nativ vorhandenen Aktivität. Dies wird vorzugsweise durch eine gegenüber dem Wildtyp reduzierte Expression des Gens für die Anthranilat-Phosphoribosyltransferase (trpD) erreicht, wobei die Expression aber nicht vollständig unterbunden ist. Dies erfolgt bevorzugt durch Einsatz einer Promotorsequenz, die gegenüber der endogen vorhandenen Promotorsequenz eine geringere Transkriptionsaktivität aufweist oder durch eine Modifikation des Abstands der Ribosomenbindestelle zum Start-Codon des trpD-Gens oder durch eine Veränderung des Start-Codons selbst. In einer bevorzugten Ausführungsform der vorliegenden Erfindung erfolgt die Reduzierung der Aktivität der Anthranilat-Phosphoribosyltransferase durch Deletion oder Inaktivierung des Gens für die endogene Anthranilat-Phosphoribosyltransferase (trpD) und den Ersatz dieses Gens durch ein Gen für eine Anthranilat-Phosphoribosyltransferase mit einer veränderten ribosomalen Bindestelle und optional einem veränderten Start-Codon wie in SEQ ID NO. 1 oder 2, bevorzugt SEQ ID NO. 2 definiert. Die Aminosäuresequenz der Anthranilat-Phosphoribosyltransferase entspricht vorzugsweise der endogenen Anthranilat-Phosphoribosyltransferase, besonders bevorzugt wird sie durch SEQ ID NO. 3 oder eine Variante davon definiert.
(ii) Deletion oder Inaktivierung des Gens, das für die Phosphoenolpyruvat-Carboxylase wie in SEQ ID NO. 4 definiert kodiert. Dies kann auf alle dem Fachmann geläufigen Weisen geschehen, vorzugsweise durch Deletion des Gens oder einer Teilsequenz davon, durch Einführung wenigstens eines Stop-Codons oder durch Deletion oder Inaktivierung der Promotorsequenz. Besonders bevorzugt ist die Deletion wenigstens eines Teils der proteinkodierenden Sequenz des Gens (SEQ ID NO. 5).

In einer noch stärker bevorzugten Ausführungsform der vorliegenden Erfindung ist die mikrobielle Zelle zusätzlich durch wenigstens eines der nachfolgend genannten Merkmale gekennzeichnet:
(iii) Erhöhte Aktivität der Shikimat-Kinase. Dies erfolgt vorzugsweise durch erhöhte Expression eines entsprechenden Enzyms. In einer Ausführungsform der vorliegenden Erfindung erfolgt die Steigerung der Aktivität durch erhöhte Expression des Gens für die endogen vorhandene Shikimat-Kinase wie in SEQ ID NO. 6 definiert oder einer Variante davon. In einer anderen bevorzugten Ausführungsform erfolgt dies durch Expression einer exogenen Shikimat-Kinase, bevorzugt wie in SEQ ID NO. 7 definiert oder einer Variante davon. Eine verstärkte Expression eines Genes kann durch alle dem Fachmann bekannten Methoden erfolgen, insbesondere durch die Einführung mehrerer Kopien des entsprechenden Gens in den Mikroorganismus oder durch die Verwendung stärkerer Promotoren zur Expression des endogen vorhandenen Enzyms. Ein besonders bevorzugter Promotor für die Expression von Fremdgenen oder die verstärkte Expression endogener Gene ist ptuf wie in SEQ ID NO. 8 definiert.
(iv) Erhöhte Aktivität der 3-Phosphoshikimat-1-carboxyvinyltransferase und Chorismat-Synthase. Bevorzugt haben diese Enzyme eine Aminosäuresequenz wie in SEQ ID NO 9 oder einer Variante davon und SEQ ID NO. 10 oder einer Variante davon definiert. Dies erfolgt bevorzugt durch die Einführung zusätzlicher Kopien der für diese Enzyme kodierenden Gene in den Mikroorganismus. Bevorzugt wird der ptuf-Promotor zur Steuerung der Expression verwendet.

Bezogen auf die Anthranilat-Phosphoribosyltransferase (SEQ ID NO. 3), Shikimat-Kinase (SEQ ID NO. 6 oder 7), 3-Phosphoshikimat-1-carboxyvinyltransferase (SEQ ID NO. 9) und Chorismat-Synthase (SEQ ID NO. 10) bedeutet "Variante" ein Enzym, das durch Hinzufügung, Deletion oder Austausch von bis zu 10 %, bevorzugt bis zu 5 %, der im jeweiligen Enzym enthaltenen Aminosäuren erhalten wird. Grundsätzlich können die vorgenannten Modifikationen an jeder beliebigen Stelle des Enzyms kontinuierlich oder diskontinuierlich erfolgen. Bevorzugt erfolgen sie aber nur am N-Terminus und/oder am C-Terminus des Polypeptids. Bei Substitutionen von Aminosäuren handelt es sich vorzugsweise um konservative Substitutionen, d.h. um solche, bei denen die veränderte Aminosäure einen Rest mit ähnlichen chemischen Eigenschaften besitzt wie die im unveränderten Enzym vorliegende Aminosäure. Es werden also besonders bevorzugt Aminosäuren mit basischen Resten gegen solche mit basischen Resten, Aminosäuren mit sauren Resten gegen solche mit ebenfalls sauren Resten, Aminosäuren mit polaren Resten gegen solche mit polaren Resten und Aminosäuren mit unpolaren gegen solche mit unpolaren Resten ausgetauscht. Bevorzugt Die spezifische Enzymaktivität einer Variante liegt vorzugsweise wenigstens bei 80 % spezifischen Aktivität des unveränderten Enzyms. Enzymtests zum Nachweis der Aktivität der vorgenannten Enzyme kann der Fachmann der Literatur entnehmen.

Die der vorliegenden Erfindung zugrunde liegende Studie hat gezeigt, dass eine 3-Desoxyarabinoheptulosanat-7-phosphat-Synthase mit einer Aminosäuresequenz wie in SEQ ID NO. 11 definiert das Wachstum der Produktionsstämme, die Substrataufnahme, die Produktbildung, sowie die Produktausbeute verbessert.

In einer weiteren bevorzugten Ausführungsform betrifft die vorliegende Erfindung eine rekombinante Zelle des Genus *Corynebacterium,* des Genus *Bacillus* oder des Genus *Pseudomonas* enthaltend ein Gen, das für eine DAHP-Synthase wie in SEQ ID NO. 11 definiert kodiert. Bevorzugt handelt es sich um eine Zelle eines Stamms von *Corynebacterium glutamicum,* stärker bevorzugt um *C. glutamicum* ATCC13032. Im Genus *Bacillus* ist *Bacillus subtilis* bevorzugt. Im Genus *Pseudomonas* ist *Pseudomonas putida* bevorzugt.

Diese Zelle enthält in einer bevorzugten Ausführungsform der vorliegenden Erfindung folgende weitere genetische Modifikationen:
(i) Eine gegenüber dem jeweiligen Wildtyp reduzierte Aktivität der Anthranilat-Phosphoribosyltransferase, wobei aber eine Restaktivät vorhanden sein muss. Die Restaktivität liegt vorzugsweise zwischen 10 % und 60 %, stärker bevorzugt zwischen 20 % und 50 % der in C. *glutamicum* ATCC13032 nativ vorhandenen Aktivität. Dies wird vorzugsweise durch eine gegenüber dem Wildtyp reduzierte Expression des Gens für die Anthranilat-Phosphoribosyltransferase (trpD) erreicht, wobei die Expression aber nicht vollständig unterbunden ist. Dies erfolgt bevorzugt durch Einsatz einer Promotorsequenz, die gegenüber der endogen vorhandenen Promotorsequenz eine geringere Transkriptionsaktivität aufweist oder durch eine Modifikation des Abstands der Ribosomenbindestelle zum Start-Codon des trpD-Gens oder durch eine Veränderung des Start-Codons selbst. In einer bevorzugten Ausführungsform der vorliegenden Erfindung erfolgt die Reduzierung der Aktivität der Anthranilat-Phosphoribosyltransferase durch Deletion oder Inaktivierung des Gens für die endogene Anthranilat-Phosphoribosyltransferase (trpD) und den Ersatz dieses Gens durch ein Gen für eine Anthranilat-Phosphoribosyltransferase mit einer veränderten ribosomalen Bindestelle und optional einem veränderten Start-Codon wie in SEQ ID NO. 1 oder 2, bevorzugt SEQ ID NO. 2 definiert. Die Aminosäuresequenz der Anthranilat-Phosphoribosyltransferase entspricht vorzugsweise der endogenen Anthranilat-Phosphoribosyltransferase, besonders bevorzugt wird sie durch SEQ ID NO. 3 oder eine Variante davon definiert.
(ii) Deletion oder Inaktivierung des Gens, das für die Phosphoenolpyruvat-Carboxylase wie in SEQ ID NO. 4 definiert kodiert. Dies kann auf alle dem Fachmann geläufigen Weisen geschehen, vorzugsweise durch Deletion des Gens oder einer Teilsequenz davon, durch Einführung wenigstens eines Stop-Codons oder durch Deletion oder Inaktivierung der Promotorsequenz. Besonders bevorzugt ist die Deletion wenigstens eines Teils der proteinkodierenden Sequenz des Gens (SEQ ID NO. 5).

In einer noch stärker bevorzugten Ausführungsform der vorliegenden Erfindung ist die mikrobielle Zelle zusätzlich durch wenigstens eines der nachfolgend genannten Merkmale gekennzeichnet:
(iii) Erhöhte Aktivität der Shikimat-Kinase. Dies erfolgt vorzugsweise durch erhöhte Expression eines entsprechenden Enzyms. In einer Ausführungsform der vorliegenden Erfindung erfolgt die Steigerung der Aktivität durch erhöhte Expression des Gens für die endogen vorhandene Shikimat-Kinase wie in SEQ ID NO. 6 definiert oder einer Variante davon. In einer anderen bevorzugten Ausführungsform erfolgt dies durch Expression einer exogenen Shikimat-Kinase, bevorzugt wie in SEQ ID NO. 7 definiert oder einer Variante davon. Eine verstärkte Expression eines Genes kann durch alle dem Fachmann bekannten Methoden erfolgen, insbesondere durch die Einführung mehrerer Kopien des entsprechenden Gens in den Mikroorganismus oder durch die Verwendung stärkerer Promotoren zur Expression des endogen vorhandenen Enzyms. Ein besonders bevorzugter Promotor für die Expression von Fremdgenen oder die verstärkte Expression endogener Gene ist ptuf wie in SEQ ID NO. 8 definiert.
(iv) Erhöhte Aktivität der 3-Phosphoshikimat-1-carboxyvinyltransferase und Chorismat-Synthase. Bevorzugt haben diese Enzyme eine Aminosäuresequenz wie in SEQ ID NO 9 oder einer Variante davon und SEQ ID NO. 10 oder einer Variante davon definiert. Dies erfolgt bevorzugt durch die Einführung zusätzlicher Kopien der für diese Enzyme kodierenden Gene in den Mikroorganismus. Bevorzugt wird der ptuf-Promotor zur Steuerung der Expression verwendet.

Alle weiter oben gegebenen Definitionen, insbesondere zu "Varianten" der Enzyme gelten auch für diese Ausführungsform.

### Verwendung einer rekombinanten mikrobiellen Zelle

In noch einer weiteren Ausführungsform betrifft die vorliegende Erfindung die Verwendung einer rekombinanten Zelle enthaltend ein Gen, das für eine DAHP-Synthase wie in SEQ ID NO. 11 definiert kodiert, zur Herstellung von oAB durch eine mikrobielle Fermentation.

Alle oben in dieser Anmeldung gegebenen Definitionen gelten auch für diese Ausführungsform. Die "Verwendung" besteht vorzugsweise in der Kultivierung der Zelle in einem geeigneten Nährmedium in Gegenwart einer fermentierbaren Kohlenstoffquelle, die von dieser Zelle in oAB umgesetzt werden kann.

In einer bevorzugten Ausführungsform der vorliegenden Erfindung wird die rekombinante Zelle zur Synthese wenigstens einer Verbindung ausgewählt aus der Gruppe bestehend aus 3-Desoxyarabinoheptulosanat-7-phosphat, 3-Dehydrochinat, 3-Dehydroxyshikimat, Shikimat, Shikimat-3-Phosphat, Chorismat, Prephenat und ortho-Aminobenzoesäure durch eine mikrobielle Fermentation verwendet.

Der Fachmann versteht, dass die metabolische und genetische Ausstattung der verwendeten mikrobiellen Zellen oder Zellen an das angestrebte angepasst werden muss, indem die Aktivität von Enzymen, welche die Umsetzung des angestrebten Produkts zu Folgeprodukten katalysieren, reduziert oder ganz unterbunden wird.

### Verfahren

In einer weiteren Ausführungsform betrifft die vorliegende Erfindung ein Verfahren enthaltend die Schritte
a) Bereitstellung einer wenigstens einer rekombinanten mikrobiellen Zelle enthaltend ein Gen, das für eine DAHP-Synthase wie in SEQ ID NO. 11 definiert kodiert;
b) Kultivierung der wenigstens einen rekombinanten mikrobiellen Zelle in einem Nährmedium, das wenigstens eine fermentierbare Kohlenstoffquelle enthält, wobei oAB produziert wird.

Alle oben gegebenen Definitionen gelten auch für diese Ausführungsform. Die "Kultivierung" der rekombinanten mikrobiellen Zellen erfolgt unter den Bedingungen, die oben in dieser Anmeldung für die "mikrobielle Fermentation" beschrieben sind. Da das Verfahren auf die effiziente Herstellung von oAB abzielt, versteht der Fachmann, dass der Begriff "wenigstens eine rekombinante mikrobielle Zelle" in dieser Ausführungsform bevorzugt eine Vielzahl von Zellen, besonders bevorzugt wenigstens 10⁹ Zellen, bedeutet.

Die "Bereitstellung" der rekombinanten mikrobiellen Zelle bedeutet, dass am Ende des Verfahrensschritts eine lebensfähige rekombinante Zelle wie oben in dieser Anmeldung beschrieben vorliegt.

Der Verfahrensschritt b) wird vorzugsweise für 15 bis 80 Stunden durchgeführt. In einer bevorzugten Ausführungsform wird der Verfahrensschritt b) solange durchgeführt, bis eine Konzentration von wenigstens 6 g/l, bevorzugt wenigstens 9 g/l oAB erreicht ist.

### Zusammensetzung

In noch einer weiteren Ausführungsform betrifft die vorliegende Erfindung eine Zusammensetzung enthaltend
a) wenigstens eine mikrobielle Zelle des Genus *Corynebacterium* enthaltend ein Gen, das für eine DAHP-Synthase wie in SEQ ID NO. 11 definiert kodiert;
b) wenigstens eine fermentierbare Kohlenstoffquelle; und
c) wenigstens eine Stickstoffquelle, wenigstens eine Phosphorquelle, wenigstens eine Schwefelquelle und Spurenelemente.

Alle oben gegebenen Definitionen gelten auch für diese Ausführungsform. Bevorzugt handelt es sich bei der erfindungsgemäßen Zusammensetzung um ein Nährmedium, das die rekombinante mikrobielle Zelle in lebensfähiger Form enthält. Die fermentierbare Kohlenstoffquelle ist eine solche, die von der mikrobiellen Zelle für die oAB-Produktion genutzt werden kann, besonders bevorzugt handelt es sich um Glucose, Xylose oder eine Mischung daraus. Der Fachmann versteht, dass der Begriff "wenigstens eine" in dieser Ausführungsform bevorzugt eine Mehrzahl von Zellen, besonders bevorzugt wenigstens 10⁹ Zellen, bedeutet, um eine hinreichende Raum-Zeit-Ausbeute der mikrobiellen Fermentation sicherzustellen.

In einer bevorzugten Ausführungsform der Erfindung enthält die erfindungsgemäße Zusammensetzung zusätzlich oAB, stärker bevorzugt wenigstens 6 g/l und noch stärker bevorzugt wenigstens 9 g/l oAB.

Die nachfolgenden Ausführungsbeispiele dienen nur dazu, die Erfindung zu illustrieren. Sie sollen den Schutzbereich der Patentansprüche in keiner Weise beschränken.

### Beispiele

### Material und Methoden

Die verwendeten Produktionsstämme und Expressionsplasmide sind in Tabelle 1 dargestellt. *C. glutamicum-Stämme* wurden aerob bei 30°C in CGXII Minimal-Medium (s. Tabelle 2) mit 2 % (w/v) Glukose als Kohlenstoffquelle und einer optischen Dichte (OD) von 1 kultiviert. Die Vorkultivierungen wurden in SY und anschließend VKII Medium durchgeführt (s. Tabelle 2). Die Hauptkultivierungen fanden entweder in 500 mL Glas-Schüttelkolben mit 30 mL Medium auf einem Rotationsschüttler mit 200 rpm statt oder in 250 mL Glas-Bioreaktoren mit 100 mL Medium in einem Fed-Batch Fermentationsprozess. Die Begasung bzw. Rührergeschwindigkeit im Bioreaktor wurde auf einen gelösten Sauerstoffgehalt (dissolved oxygen, DO) von 30 % eingestellt. Zu Beginn der Fermentation wurden 20 g/L Glukose vorgelegt. Bei Überschreitung des DO im Medium von 37 % wurde die Zugabe von Glukose durch eine Bolus-Dosierung auf eine Konzentration von 20 g/L Glukose gelöst. Zur pH-Kontrolle wurde ein Gemisch aus 1,5 mol/L NaOH und 4,9 mol/L (NH₄)₂OH zudosiert.

Für die Analyse des Wachstums wurden OD Messungen bei einer Wellenlänge von 600 nm durchgeführt. Zusätzlich wurde das Trockengewicht der Zellen bestimmt. Der Überstand der Proben diente zur Quantifizierung der Glukose am CedexBio Analyzer und zusätzlich zur Messung der Produkt-Konzentration im Medium mittels HPLC Analyse an einer Luna 3 µm C18(2) Trennsäule.

**Tabelle 1: Für dieses Patent verwendete Produktionsstämme und Expressionsplasmide**

| **Stamm oder Plasmid** | **Beschreibung** |
|---|---|
| | |

| **Stamm** | |
|---|---|
| *C. glutamicum Δppc AtrpD::trpD5* | Derivat von ATCC13032 mit funktioneller Deletion der Phosphoenolpyruvat-Carboxylase (SEQ ID NO. 4) und genomischem Austausch von *trpD* durch SEQ ID NO. 1 |
| *C. glutamicum Δppc ΔtrpD::trpD5 ΔaroF* | Derivat von ATCC13032 mit funktioneller Deletion der Phosphoenolpyruvat-Carboxylase (SEQ ID NO. 4) und *aroF* sowie genomischem Austausch von *trpD* durch SEQ ID NO. 1 |
| | |

| **Plasmid** | |
|---|---|
| pEKEx2 | Kan^{R}; *C. glutamicum* / *E. coli* Shuttle-Vektor für regulierbare Genexpression (P*_{tac} lacl^{Q}* pBL1 *oriV_{Cg}* pUC18 *oriV_{Ec}*) |
| pEKEx2_*aroF*^{wt} | Kan^{R}; pEKEx2 Derivat, welches das native *aroF* Gen (SEQ ID NO. 17) aus *C. glutamicum* unter Kontrolle des *tac* Promotors trägt |
| pEKEx2_*aroF*^{E154N} | Kan^{R}; pEKEx2 Derivat, welches eine Genvariante des *aroF* Gens aus *C. glutamicum* mit Codonaustausch für den Aminosäureaustausch |
| | E154N (SEQ ID NO. 18) unter Kontrolle des *tac* Promotors trägt |
| pEKEx2_*aroG*^{new} | Kan^{R}; pEKEx2 Derivat, welches eine Genvariante des *aroG* Gens aus *E. coli* mit Codonaustausch für den Aminosäureaustausch L76V und S211F (SEQ ID NO. 11) unter Kontrolle des *tac* Promotors trägt |

**Tabelle 2: Zusammensetzung von Vor- und Hauptkultivierungsmedien sowie Stammlösungen**

| *SY Medium für Vorkultivierungen von Schüttelkolben und Fermentationen* | | |
|---|---|---|
| **Komponente** | **Konzentration** | **Kommentar** |
| Soja Pepton | 16 g/L | - |
| Hefeextrakt | 10 g/L | - |
| NaCl | 5 g/L | - |
| pH mit KOH auf 7,0 einstellen und autoklavieren | | |
| Glukose*H2O | 15 g/L | Zugabe nach Autoklavieren |
| | | |

| *VKII Medium für Vorkultivierungen von Schüttelkolben und Fermentationen* | | |
|---|---|---|
| **Komponente** | **Konzentration** | **Kommentar** |
| KH₂PO₄ | 1 g/L | - |
| K₂HPO₄ | 1 g/L | - |
| (NH₄)₂SO₄ | 20 g/L | - |
| CaCl₂*2H₂O | 0,01 g/L | - |
| MgSO₄*7H₂O | 0,25 g/L | - |
| MOPS | 42 g/L | - |
| UREA | 5 g/L | - |
| Hefeextrakt | 5 g/L | - |
| pH mit KOH auf 7,0 einstellen und autoklavieren | | |
| Spurenelement-Stammlösung (1000x) | 1 mL/L | Zugabe nach Autoklavieren |
| Biotin | 0,002 g/L | - |
| Glukose*H2O | 40 g/L | - |
| | | |

| *CGXII Minimal-Medium für Schüttelkolben Kultivierungen* | | |
|---|---|---|
| **Komponente** | **Konzentration** | **Kommentar** |
| KH₂PO₄ | 1 g/L | - |
| K₂HPO₄ | 1 g/L | - |
| (NH₄)₂SO₄ | 10 g/L | - |
| MOPS | 62 g/L | - |
| CaCl₂*2H₂O | 0,01 g/L | - |
| MgSO₄*7H₂O | 0,25 g/L | - |
| pH mit KOH auf 7,0 einstellen und autoklavieren | | |
| Spurenelement-Stammlösung (1000x) | 1 mL/L | Zugabe nach Autoklavieren |
| Biotin | 0,002 g/L | - |
| Glukose*H2O | 20 g/L | - |
| | | |
| | | |

| *CGXII Minimal-Medium für Fermentationen im Bioreaktor* | | |
|---|---|---|
| **Komponente** | **Konzentration** | **Kommentar** |
| KH₂PO₄ | 2 g/L | - |
| K₂HPO₄ | 2 g/L | - |
| (NH₄)₂SO₄ | 5 g/L | - |
| MOPS | 62 g/L | - |
| CaCl₂*2H₂O | 0,02 g/L | - |
| MgSO₄*7H₂O | 1 g/L | - |
| pH mit KOH auf 7,0 einstellen und autoklavieren | | |
| Spurenelement-Stammlösung (1000x) | 1 mL/L | Zugabe nach Autoklavieren |
| Biotin | 0,002 g/L | - |
| Polypropylenglykol | 0,005 % v/v | - |
| Glukose*H2O | 20 g/L | - |
| | | |

| *Zusammensetzung der verwendeten Spurenelement-Stammlösung (1000x)* | | |
|---|---|---|
| **Komponente** | **Konzentration** | |
| CuSO₄*5H₂O | 0,2 g/L | |
| ZnSO₄*7H₂O | 1 g/L | |
| NiCl₂*6H₂O | 0,02 g/L | |
| MnSO₄*H₂O | 10 g/L | |
| FeSO₄*7H₂O | 10 g/L | |

### Ergebnisse

Es wurden Schüttelkolbenkultivierungen mit dem Produktionsstamm *C. glutamicum Δppc ΔtrpD::trpD5* durchgeführt, der mit unterschiedlichen Expressionsplasmiden transformiert wurde. In Tabelle 3 sind Produkttiter gezeigt, die jeweils über den Verlauf einer Kultivierungszeit von 48 h gemessen wurden. Es ist zu erkennen, dass sowohl die Leerplasmid-Kontrolle, als auch *aroF*^{wt} bzw. *aroF*^{E154N} tragende Expressionsplasmide keinen Effekt auf die Produktbildung im Stammhintergrund zeigten. Ausschließlich der Produktionsstamm mit *aroG*^{new} Expressionsplasmid zeigte eine signifikante Steigerung des finalen Titers um 54 - 72 % (s. Tabelle 3).

**Tabelle 3: Produktkonzentrationen (g/L) aus Schüttelkolbenkultivierungen mit unterschiedlichen Expressionsplasmiden. Die gemessenen Werte sind Mittelwerte aus zwei biologischen Replikaten. Messpunkte mit einer Standardabweichung über 5 % wurden mit einem * markiert.**

| **Produktionsstamm** | **Kultivierungszeit (h)** | | | | | |
|---|---|---|---|---|---|---|
| | **5** | **7** | **9** | **12** | **24** | **48** |
| *C. glutamicum Δppc AtrpD::trpD5* pEKEx2 | 0,018 | 0,056 | 0,111 | 0,215 | 0,612 | 0,551* |
| *C. glutamicum Δppc* Δ*trpD::trpD5* pEKEx2_*aroF*^{wt} | 0,002 | 0,018 | 0,066* | 0,188* | 0,616 | 0,615 |
| *C. glutamicum* Δ*ppc* Δ*trpD::trpD5* pEKEx2_*aroF*^{E154N} | 0,002* | 0,024* | 0,067 | 0,159* | 0,586 | 0,557* |
| *C. glutamicum* Δ*ppc* Δ*trpD::trpD5* pEKEx2_*aroG^{new}* | 0,043 | 0,087 | 0,138 | 0,244 | 0,871 | 0,947 |

Um den positiven Effekt der Genvariante *aroG*^{new} genauer zu charakterisieren, wurden Fermentationen durchgeführt, in denen der Produktionsstamm *C. glutamicum* Δ*ppc* Δ*trpD::trpD5* Δ*aroF* jeweils mit den in Tabelle 3 gezeigten Expressionsplasmiden transformiert wurde. Wie in Tabelle 4 gezeigt, wirkt sich die Transformation mit dem *aroG*^{new} tragenden Expressionsplasmid positiv auf das Wachstum der Zellen, die Substrataufnahme, Produkttiter sowie Produktausbeuten aus (s. Tabelle 4). Bezogen auf die finale Produktausbeute erzielte die Ausprägung von AroG^{new} eine Verbesserung um 44 - 48 %.

**Tabelle 4: Fermentative Charakterisierung eines Produktionsstammes mit unterschiedlichen Expressionsplasmiden im Bioreaktor.**

| ***C. glutamicum* Δ*ppc* Δ*trpD::trpD5* Δ*aroF* pEKEx2** | | | | |
|---|---|---|---|---|
| | | | | |

| **Zeit (h)** | **Trockengewicht (g)** | **Glukose-Verbrauch (g)** | **oAB (g/L)** | **Produktausbeute (g oAB/g Glukose)** |
|---|---|---|---|---|
| 0,0 | -0,02 | 0,00 | 0,00 | 0,000 |
| 5,6 | 0,02 | 0,20 | 0,05 | 0,022 |
| 20,6 | 0,11 | 0,68 | 0,53 | 0,071 |
| 24,2 | 0,19 | 0,93 | 0,62 | 0,061 |
| 28,9 | 0,38 | 1,46 | 1,19 | 0,073 |
| 46,1 | 1,54 | 4,99 | 4,32 | 0,084 |
| 49,9 | 1,75 | 5,18 | 1,99 | 0,044 |
| 53,8 | 2,09 | 6,72 | 4,40 | 0,067 |
| 68,8 | 4,38 | 12,70 | 4,38 | 0,041 |
| 70,6 | 4,76 | 13,67 | 4,57 | 0,041 |
| | | | | |

| ***C. glutamicum* Δ*ppc* Δ*trpD::trpD5* Δ*aroF* pEKEx2-*aroF*^{wt}** | | | | |
|---|---|---|---|---|
| | | | | |

| **Zeit (h)** | **Trockengewicht (g)** | **Glukose-Verbrauch (g)** | **oAB (g/L)** | **Produktausbeute (g oAB/g Glukose)** |
|---|---|---|---|---|
| 0,0 | -0,02 | 0,00 | 0,01 | 0,000 |
| 5,6 | 0,01 | 0,15 | 0,06 | 0,040 |
| 20,6 | 0,08 | 0,62 | 0,57 | 0,085 |
| 24,2 | 0,16 | 0,88 | 0,66 | 0,069 |
| 28,9 | 0,35 | 1,32 | 1,47 | 0,100 |
| 46,1 | 1,40 | 4,56 | 4,89 | 0,103 |
| 49,9 | 1,62 | 5,37 | 2,85 | 0,054 |
| 53,8 | 1,89 | 6,76 | 4,84 | 0,072 |
| 68,8 | 3,86 | 12,50 | 4,44 | 0,042 |
| 70,6 | 4,52 | 13,87 | 4,68 | 0,040 |

| ***C. glutamicum* Δ*ppc* Δ*trpD::trpD5* Δ*aroF* pEKEx2-*aroF*^{E154N}** | | | | |
|---|---|---|---|---|
| | | | | |

| **Zeit (h)** | **Trockengewicht (g)** | **Glukose-Verbrauch (g)** | **oAB (g/L)** | **Produktausbeute (g oAB/g Glukose)** |
|---|---|---|---|---|
| 0,0 | -0,05 | 0,00 | 0,00 | 0,000 |
| 5,6 | 0,00 | 0,17 | 0,04 | 0,023 |
| 20,6 | 0,12 | 0,68 | 0,49 | 0,066 |
| 24,2 | 0,23 | 0,94 | 0,71 | 0,068 |
| 28,9 | 0,47 | 1,50 | 1,47 | 0,089 |
| 46,1 | 1,48 | 4,76 | 4,23 | 0,085 |
| 49,9 | 1,64 | 5,61 | 3,87 | 0,068 |
| 53,8 | 1,98 | 6,46 | 4,86 | 0,076 |
| 68,8 | 3,94 | 12,54 | 4,45 | 0,041 |
| 70,6 | 4,62 | 13,42 | 4,60 | 0,040 |
| | | | | |

| ***C. glutamicum* Δ*ppc* Δ*trpD::trpD5* Δ*aroF* pEKEx2-*aroG*^{new}** | | | | |
|---|---|---|---|---|
| | | | | |

| **Zeit (h)** | **Trockengewicht (g)** | **Glukose-Verbrauch (g)** | **oAB (g/L)** | **Produktausbeute (g oAB/g Glukose)** |
|---|---|---|---|---|
| 0,0 | -0,03 | 0,00 | 0,00 | 0,000 |
| 5,6 | 0,04 | 0,17 | 0,07 | 0,036 |
| 20,6 | 0,36 | 1,05 | 1,26 | 0,111 |
| 24,2 | 0,56 | 1,59 | 1,90 | 0,110 |
| 28,9 | 0,94 | 2,34 | 3,88 | 0,157 |
| 46,1 | 2,22 | 6,63 | 6,13 | 0,094 |
| 49,9 | 2,62 | 7,88 | 6,06 | 0,081 |
| 53,8 | 3,05 | 9,35 | 8,46 | 0,099 |
| 68,8 | 6,45 | 18,03 | 8,46 | 0,061 |
| 70,6 | 7,05 | 20,17 | 8,96 | 0,059 |

### Diskussion

Die beschriebenen Versuche zeigen, dass die Einbringung der *aroG*^{new} Genvariante einen überraschenden Vorteil darstellt für das Wachstum der Produktionsstämme, die Substrataufnahme, die Produktbildung, sowie für die Produktausbeute. Diese Beobachtungen waren allein auf die Modifikationen der *E. coli*-basierten *aroG* Genvariante im *C. glutamicum* Stammhintergrund zurückzuführen und konnten nicht mit weiteren untersuchten Varianten von DAHP Synthetasen repliziert werden.

## Patentansprüche

1. Verwendung einer 3-Desoxyarabinoheptulosanat-7-phosphat-Synthase (DAHP-Synthase) mit einer Aminosäuresequenz wie durch SEQ ID NO. 11 definiert oder einer Variante davon zur Herstellung von ortho-Aminobenzoesäure (oAB) durch eine mikrobielle Fermentation.

2. Rekombinante Zelle des Genus *Corynebacterium* enthaltend ein Gen, das für eine DAHP-Synthase wie in SEQ ID NO. 11 definiert kodiert.

3. Die rekombinante Zelle gemäß Anspruch 2, wobei es sich um *C. glutamicum* handelt.

4. Die rekombinante Zelle gemäß Anspruch 2 oder, zusätzlich **gekennzeichnet durch**
(i) Eine gegenüber dem jeweiligen Wildtyp reduzierte Aktivität der Anthranilat-Phosphoribosyltransferase, wobei aber eine Restaktivät vorhanden sein muss; und
(ii) Deletion oder Inaktivierung des Gens, das für die Phosphoenolpyruvat-Carboxylase wie in SEQ ID NO. 4 definiert kodiert.

5. Verwendung einer rekombinanten Zelle enthaltend ein Gen, das für eine DAHP-Synthase wie in SEQ ID NO. 11 definiert kodiert, zur Herstellung von oAB durch eine mikrobielle Fermentation.

6. Die Verwendung nach Anspruch 5, wobei die mikrobielle Zelle ein *Corynebacterium* sp. ist.

7. Die Verwendung der rekombinanten Zelle nach Anspruch 4 oder 5, wobei die rekombinante Zelle sich vom Wildtyp des betreffenden Mikroorganismus zusätzlich durch folgende Merkmale unterscheidet:
(i) Eine gegenüber dem jeweiligen Wildtyp reduzierte Aktivität der Anthranilat-Phosphoribosyltransferase, wobei aber eine Restaktivät vorhanden sein muss; und
(ii) Deletion oder Inaktivierung des Gens, das für die Phosphoenolpyruvat-Carboxylase wie in SEQ ID NO. 4 definiert kodiert.

8. Verfahren enthaltend die Schritte
a) Bereitstellung einer wenigstens einer rekombinanten mikrobiellen Zelle enthaltend ein Gen, das für eine DAHP-Synthase wie in SEQ ID NO. 11 definiert kodiert;
b) Kultivierung der wenigstens einen rekombinanten mikrobiellen Zelle in einem Nährmedium, das wenigstens eine fermentierbare Kohlenstoffquelle enthält, wobei oAB produziert wird.

9. Zusammensetzung enthaltend
a) wenigstens eine rekombinante mikrobielle Zelle enthaltend ein Gen, das für eine DAHP-Synthase wie in SEQ ID NO. 11 definiert kodiert;
b) wenigstens eine fermentierbare Kohlenstoffquelle; und
c) wenigstens eine Stickstoffquelle, wenigstens eine Phosphorquelle, wenigstens eine Schwefelquelle und Spurenelemente.

10. Zusammensetzung gemäß Anspruch 9, zusätzlich enthaltend oAB.
